# FASCICULE DE BREVET EUROPEEN

(11) **EP 0 825 151 B1**
(45) Date de publication et mention de la délivrance du brevet: **19.12.2001**
(21) Numéro de dépôt: 97401943.2
(22) Date de dépôt: 18.08.1997
(51) Int. Cl.: C01B 39/02, B01J 29/70

(54) **Zéolithe modifiée de type structural NES, et son utilsation en dismutation et/ou transalkylation d'hydrocarbures alkylaromatiques**
Modifizierter Zeolith mit NES Struktur, und seine Verwendung für die Dismutation und/oder Transalkylierung von alkylaromatischen Kohlenwasserstoffen
Modified zeolite with the NES structure, and the use thereof for the dismutation and/or transalkylation of alkylaromatic hydrocarbons

(30) Priorité: 23.08.1996 FR 9610508
(43) Date de publication de la demande: 25.02.1998
(73) Titulaire: INSTITUT FRANCAIS DU PETROLE, 92852 Rueil-Malmaison Cedex (FR)
(72) Inventeur: Casci, John Leonello, Cleveland TS23 1LB (GB); Chouteau, Nicolas, 92000 Nanterre (FR); Thompson, Nicolas Edward, Clitheroe, Lancashire BB7 1NP (GB); Rebours, Bernadette, 95120 Ermont (FR); Benazzi, Eric, 78360 Montesson (FR); Alario, Fabio, 92200 Neuilly sur Seine (FR)

(56) Documents cités:
- EP-A- 0 377 291
- EP-A- 0 378 916
- EP-A- 0 488 867
- EP-A- 0 601 924

## Description

L'invention concerne une zéolithe modifiée de type structural NES, de préférence la Nu-87, comprenant du silicium et au moins un élément T choisi dans le groupe formé par l'aluminium, le fer, le gallium, le bore, et dans laquelle une partie au moins de l'élément T a été ôtée de façon à ce que le rapport Si/T atomique global soit supérieur à 25. L'extraction d'élément T de la charpente (et plus particulièrement de l'aluminium, et plus spécifiquement dans la NU-87) peut être réalisée de différentes manières, par exemple par au moins un traitement thermique, éventuellement en présence de vapeur d'eau, suivi d'au moins une attaque acide par au moins une solution d'un acide minéral ou organique, ou bien par une attaque acide directe par au moins une solution d'un acide minéral ou organique. L'invention concerne également un support de catalyseur ainsi qu'un catalyseur comprenant ladite zéolithe. L'invention concerne aussi l'utilisation d'un tel catalyseur en dismutation et/ou transalkylation d'hydrocarbures alkylaromatiques, tel que le toluène et les composés aromatiques à au moins 9 atomes de carbone par molécule.

De nombreux catalyseurs de dismutation et/ou de transalkylation ont déjà été décrits dans l'art antérieur, à base de mordénite (US-A-3.506.731, US-A-4.151.120, US-A-4.180.693, US-A-4.210.770, US-A-3.281.483, US-A-3.780.121, ou US-A-3.629.351, ou bien aussi à base de zéolithe oméga (US-A-5.210.356, US-A-5.371.311).

Plus récemment encore, il a été décrit dans le brevet EP-B1-0.378.916, que la zéolithe NU-87, qui est une zéolithe de type structural NES, peut être utilisée en tant que catalyseur pour la réaction de dismutation du toluène.

De façon surprenante, une zéolithe désaluminée de type structural NES, de préférence la zéolithe NU-87, au moins en partie, de préférence pratiquement totalement, sous forme acide, et comportant un rapport Si/T supérieur à environ 25 conduit, lors de son incorporation dans un catalyseur, à des performances catalytiques, et en particulier à des activités, stabilités et sélectivités, améliorées dans les réactions de dismutation d'hydrocarbures alkylaromatiques tel que le toluène, et/ou de transalkylation d'hydrocarbures alkylaromatiques tels que le toluène et les alkylaromatiques à au moins 9 atomes de carbone, tels que les triméthyl benzènes, par rapport aux catalyseurs de l'art antérieur,. et en particulier par rapport aux zéolithes NU-87 non désaluminées décrites dans le brevet européen EP-B1-0.378.916, ainsi qu'il sera démontré ci-après dans les exemples.

L'invention concerne une zéolithe modifiée de type structural NES, de préférence une zéolithe NU-87, comprenant du silicium et au moins un élément T choisi dans le groupe formé par l'aluminium, le fer, le gallium, et le bore, de préférence l'aluminium, dans laquelle une partie au moins d'élément T a été ôtée (de préférence elle a été désaluminée) et elle possède un rapport Si/T atomique global supérieur à 25, de manière préférée compris entre 25 et 350, et avantageusement entre 25 et 150.

L'invention concerne aussi un support de catalyseur et un catalyseur comportant au moins ladite zéolithe de type structural NES, de préférence ladite zéolithe NU-87. Ladite zéolithe, et de préférence la NU-87, est désaluminée et au moins en partie, de préférence pratiquement totalement, sous forme acide, comprenant du silicium et au moins un élément T choisi dans le groupe formé par l'aluminium, le fer, le gallium, et le bore, de préférence l'aluminium, et au moins une matrice (ou liant). Le rapport Si/T atomique global de ladite zéolithe, en particulier désaluminée, est généralement supérieur à 25, de preférence supérieur à 30, et de manière encore plus préférée compris entre 25 et environ 350 ou encore entre environ 25 et environ 150, ledit catalyseur comprend aussi éventuellement au moins un élément choisi dans l'ensemble formé par les groupes IB et VIII de la classification périodique des éléments, de préférence choisi dans le groupe formé par Ag, Ni et Pt, de préférence Ni ou Pt.

La matrice est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium, les silice-alumines et le charbon, de préférence parmi les éléments du groupe formé par les alumines et les argiles.

La zéolithe de type structural NES possède un réseau microporeux bidimensionnel, dont le diamètre des pores est de 4,7 x 6,0 Å ("Atlas of Zeolites Structure Types", W.M. Meier et D.H. Olson, 3rd Edition, 1992). Elle est généralement choisie dans le groupe formé par la zéolithe Nu-87 et la zéolithe SSZ-37, et de préférence elle est la zéolithe Nu-87. Les diagrammes de diffraction des rayons X de la zéolithe de type structural NES sont donnés dans le brevet européen EP-B1-0.378.916, et dans le brevet européen EP-B1-0.377.291. Ainsi qu'il est connu de l'homme du métier, une zéolithe de type structural NES possède les raies principales de diffraction des rayons X de sa structure, mais l'intensité de ces raies est susceptible de varier en fonction de la forme sous laquelle se trouve ladite zéolithe, sans que cela pose néanmoins de doutes sur l'appartenance de ladite zéolithe à ladite structure. Ainsi la zéolithe désaluminée de structure NES selon l'invention possède les raies principales de sa structure telles qu'elles sont données par le brevet européen EP-B1-0.378.916, avec une intensité des raies susceptible d'être différente de ce qui est indiqué dans ledit brevet.

Lorsqu'elle est comprise dans le catalyseur selon l'invention, la zéolithe de type structural NES est au moins en partie, de préférence pratiquement totalement, sous forme acide, c'est-à-dire sous forme hydrogène (H⁺). Le rapport atomique Na/T est généralement inférieur à 0,45 et de préférence inférieur à 0,30 et de manière encore plus préférée inférieur à 0,15. On peut même atteindre des valeurs inférieures à 0,1, de préférence à 0,05 ou encore à 0, 01.

Le catalyseur selon l'invention contient généralement de 10 à 99%, et de préférence de 20 à 95%, de zéolithe de type structural NES au moins en partie sous forme acide. Dans le cas où le catalyseur de la présente invention contient au moins un élément choisi dans l'ensemble formé par les groupes IB et VIII de la classification périodique des éléments, la teneur pondérale du(des)dit(s) élément(s) est généralement comprise entre 0,01 et 10%, de préférence entre 0,05 et 7%, et de façon encore plus préférée entre 0,10 et 5%. Le complément à 100 % poids consiste généralement en la part de matrice dans le catalyseur.

Le rapport T/Al global de la zéolithe ainsi que la composition chimique des échantillons sont déterminés par fluorescence X et absorption atomique.

A partir des diagrammes de diffraction des rayons X, on mesure, pour chaque échantillon, la surface totale du signal sur une plage angulaire (2θ) de 6 à 40°, puis, dans la même zone, la surface des raies en nombre d'impulsions pour un enregistrement pas à pas de 3 secondes avec des pas de 0,02°(2θ). Le rapport de ces deux valeurs, Surface des raies/Surface totale, est caractéristique de la quantité de matière cristallisée dans l'échantillon. On compare ensuite ce rapport ou "taux de pics", pour chaque échantillon traité, au taux de pics d'une référence étalon considérée arbitrairement comme totalement (100%) cristallisée. Le taux de cristallinité est donc exprimé en pourcentage par rapport à une référence, qu'il importe de bien choisir, car l'intensité relative des raies varie en fonction de la nature, de la proportion et de la position des différents atomes dans l'unité de structure, et en particulier des cations et du structurant. Dans le cas des mesures effectuées dans les exemples de la présente description, la référence choisie est la forme calcinée sous air sec et échangée 3 fois, successivement, par une solution de nitrate d'ammonium de la zéolithe NU-87.

Une caractérisation plus fine de la zéolithe modifiée de type structural NES selon l'invention est susceptible d'être donnée, ainsi qu'il est fait ci-après et qu'il est repris dans les exemples. Ainsi, les zéolithes de type NU-87 cristallisant dans le système monoclinique, quatre paramètres sont donc à mesurer pour caractériser la géométrie de la maille élémentaire (valeurs typiques qui sont sensiblement les suivantes, étant donné qu'il est connu de l'homme du métier que de légères variations sont possibles selon la forme que prend ladite zéolithe : a= 14,3 Å, b=22,4 Å, c= 25,1 Å et β= 151,5°). Par ailleurs, cette maille est grande et peu symétrique, les diffractogrammes des zéolithes de type NU-87 comportent donc des raies nombreuses qui interfèrent largement. La structure de ces solides a été résolue et on dispose des positions atomiques de la charpente (M.D.Shannon, J.L.Casci, P.A.Cox and S.J.Andrews, Structure of the two-dimensinal medium-pore high-silica zeolite NU-87, Letters to Nature, vol.353, 1991). On a donc utilisé l'affinement complet de diagramme par la méthode de Rietveld pour obtenir une mesure fiable des paramètres de maille. Le logiciel GSAS (General Structure Analysis System) a été utilisé, et les données introduites, outre le système cristallin monoclinique et des paramètres a, b, c et β de départ comportent : le groupe d'espace P2₁/c, les positions atomiques, taux d'occupation des sites et facteurs de température de l'unité asymétrique (M.D.Shannon, J.L.Casci, P.A.Cox and S.J.Andrews, Structure of the two-dimensinal medium-pore high-silica zeolite NU-87, Letters to Nature, vol.353, 1991) et les paramètres instrumentaux qui déterminent les profils de raies. Les paramètres d'affinement sont donc a, b, c, β et le volume de la maille cristalline.

La plage angulaire utilisée est de 15 à 70 °(2θ), car les raies situées aux plus grands angles permettent d'obtenir plus de précision sur la mesure des distances interéticulaires et donc sur les paramètres de maille.

Il est aussi possible d'estimer le volume microporeux à partir de la quantité d'azote adsorbée à 77 K pour une pression partielle P/Po égale à 0,19, à titre indicatif.

L'invention concerne aussi la préparation de ladite zéolithe désaluminée de type structural NES et dudit catalyseur.

Pour préparer la zéolithe désaluminée de type structural NES selon l'invention, dans le cas préféré où T est Al, deux méthodes de désalumination peuvent être employées, à partir de la zéolithe de type structural NES brute de synthèse comprenant du structurant organique. Elles sont décrites ci-après. Mais toute autre méthode connue de l'homme du métier entre aussi dans le cadre de l'invention.

Ces méthodes décrites pour Al peuvent également être valables pour les autres éléments T.

La première méthode dite de l'attaque acide directe comprend une première étape de calcination sous flux d'air sec, à une température généralement comprise entre environ 450 et 550°C, qui a pour but d'éliminer le structurant organique présent dans la microporosité de la zéolithe, suivie d'une étape de traitement par une solution aqueuse d'un acide minéral tel que HNO₃ ou HCI ou organique tel que CH₃CO₂H. Cette dernière étape peut être répétée autant de fois qu'il est nécessaire afin d'obtenir le niveau de désalumination voulue. Entre ces deux étapes il est possible de réaliser un ou plusieurs échanges ioniques par au moins une solution NH₄NO₃, de manière à éliminer au moins en partie, de préférence pratiquement totalement, le cation alcalin, en particulier le sodium. De même, à la fin du traitement de désalumination par attaque acide directe, il est éventuellement possible de réaliser un ou plusieurs échanges ioniques par au moins une solution NH₄NO₃, de manière à éliminer les cations alcalins résiduels et en particulier le sodium.

Pour atteindre le rapport Si/Al désiré, il est nécessaire de bien choisir les conditions opératoires ; de ce point de vue les paramètres les plus critiques sont la température de traitement par la solution aqueuse d'acide, la concentration dudit acide, la nature dudit acide, le rapport entre la quantité de solution acide et la masse de zéolithe traitée, la durée de traitement et le nombre de traitement réalisés.

On peut également effectuer des traitements de désalumination par des composés chimiques désaluminants tels que (à titre d'exemples et de façon non exhaustive) le tétrachlorure de silicium (SiCl₄), l'hexafluorosilicate d'ammonium [(NH4)₂SiF₆], l'acide éthylènediaminetétracétique (EDTA) ainsi que sa forme mono et disodique. Ces réactifs peuvent être utilisés en solution ou en phase gaz par exemple dans le cas de SiCl₄.

La deuxième méthode dite de traitement thermique (en particulier à la vapeur d'eau ou "steaming") + attaque acide, comprend, dans un premier temps, la calcination sous flux d'air sec, à une température généralement comprise entre environ 450 et 550°C, qui a pour but d'éliminer le structurant organique occlus dans la microporosité de la zéolithe. Puis le solide ainsi obtenu est soumis à un ou plusieurs échanges ioniques par au moins une solution NH₄NO₃, de manière à éliminer au moins en partie, de préférence pratiquement totalement, le cation alcalin, en particulier le sodium, présent en position cationique dans la zéolithe. La zéolithe ainsi obtenue est soumise à au moins un cycle de désalumination de charpente, comportant au moins un traitement thermique réalisé, éventuellement et de préférence en présence de vapeur d'eau, à une température généralement comprise entre 550 et 900°C, et éventuellement suivie d'au moins une attaque acide par une solution aqueuse d'un acide minéral ou organique. Les conditions de calcination en présence de vapeur d'eau (température, pression de vapeur d'eau et durée du traitement) ainsi que les conditions d'attaque acide post-calcination (durée de l'attaque, concentration de l'acide, nature de l'acide utilisé et le rapport entre le volume d'acide et la masse de zéolithe), sont adaptées de manière à obtenir le niveau de désalumination souhaité. Dans le même but on peut aussi jouer sur le nombre de cycles traitement thermique-attaque acide qui sont effectués.

Une variante de cette deuxième méthode peut constituer à remplacer l'étape dite de l'attaque acide, c'est-à-dire du traitement par une solution d'acide, par un traitement par une solution d'un composé chimique désaluminant tels que, par exemple, ceux cités précédemment à savoir le tétrachlorure de silicium (SiCl₄), l'hexafluorosilicate d'ammonium [(NH4)₂SiF₆], l'acide éthylènediaminetétracétique (EDTA) ainsi que sa forme mono et disodique.

Dans le cas préféré où T est Al, le cycle de désalumination de la charpente, comportant au moins une étape de traitement thermique réalisé, éventuellement et de préférence, en présence de vapeur d'eau et au moins une étape d'attaque en milieu acide de la zéolithe de type structural NES, peut être répété autant de fois qu'il est nécessaire pour obtenir la zéolithe de type structural NES désaluminée possédant les caractéristiques désirées. De même, suite au traitement thermique réalisé, éventuellement et de préférence en présence de vapeur d'eau, plusieurs attaques acides successives, avec des solutions en acide de concentrations différentes, peuvent être opérées.

Une variante de cette deuxième méthode de calcination peut consister à réaliser le traitement thermique de la zéolithe de type structural NES contenant le structurant organique, à une température généralement comprise entre 550 et 850°C, éventuellement et de préférence en présence de vapeur d'eau. Dans ce cas les étapes de calcination du structurant organique et de désalumination de la charpente sont réalisées simultanément. Puis, la zéolithe est éventuellement traitée par au moins une solution aqueuse d'un acide minéral (par exemple de HNO₃ ou de HCI) ou organique(CH₃CO₂H par exemple). Enfin, le solide ainsi obtenu peut être éventuellement soumis à au moins un échange ionique par au moins une solution NH₄NO₃, de manière à éliminer pratiquement tout cation alcalin, en particulier le sodium, présent en position cationique dans la zéolithe.

La préparation du catalyseur peut être effectuée selon toute méthode connue de l'homme du métier. En général, elle est obtenue par mélange de la matrice et de la zéolithe puis par mise en forme. L'élément éventuel de l'ensemble formé par les groupes lB et VIII de la classification périodique des éléments peut être introduit soit avant la mise en forme, ou bien lors du mélange, ou bien sur la zéolithe elle même avant de la mélanger, ou bien, et de préférence, après la mise en forme. On comprend ainsi que mélange matrice + zéolithe est un support pour le catalyseur contenant l'élément des groupes IB et VIII. Ce mélange peut également agir comme un catalyseur. La mise en forme est généralement suivie d'une calcination, généralement à une température comprise entre 250 et 600 °C. L'élément éventuel de l'ensemble formé par les groupes IB et VIII de la classification périodique des éléments peut être introduit après ladite calcination. Dans tous les cas, ledit élément est généralement déposé au choix soit, de préférence, pratiquement totalement sur la zéolithe, soit pratiquement totalement sur la matrice, soit en partie sur la zéolithe et en partie sur la matrice, ce choix s'effectuant de la manière qui est connue de l'homme du métier, par les paramètres utilisés lors dudit dépôt, comme par exemple la nature du précurseur choisi pour effectuer ledit dépôt.

L'élément des groupes IB ou VIII, de préférence choisi dans le groupe formé par Ag, Ni et Pt, de préférence Ni ou Pt, peut également être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Le dépôt éventuel d'élément (des éléments) des groupes IB et VIII est suivi en général d'une calcination sous air ou oxygène, généralement entre 300 et 600°C, de préférence entre 350 et 550°C, et pour une durée comprise entre 0,5 et 10 heure(s), de préférence entre 1 et 4 heure(s).

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur selon l'invention est généralement telle que le catalyseur est de préférence sous forme de pastilles, d'aggrégats, d'extrudés ou de billes, en vue de son utilisation.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250°C, de préférence entre 40 et 200°C. Ladite étape de séchage est de préférence menée pendant la montée en température nécessaire pour effectuer ladite calcination.

On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures, de façon à obtenir l'élément des groupes IB et VIII principalement sous forme réduite nécessaire à l'activité catalytique. Une telle réduction peut avoir lieu ex situ ou in situ, par rapport au lieu d'utilisation dudit catalyseur dans une réaction donnée.

Le catalyseur contenant la zéolithe modifiée selon l'invention, et en particulier la NU-87, est utilisé pour la conversion d'hydrocarbures.

L'invention concerne en particulier l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et d'hydrocarbures alkylaromatiques généralement en C₉⁺ (c'est à dire à au moins 9 atomes de carbone par molécule), avec des mélanges toluène-AC₉⁽⁺⁾ (où AC₉⁽⁺⁾ désigne les hydrocarbures alkylaromatiques comportant au moins 9 atomes de carbone par molécule) pouvant contenir de 0 à 100% de AC₉⁽⁺⁾ par rapport au mélange total, telle que par exemple la transalkylation du toluène et de triméthylbenzènes pour produire des xylènes. Ledit catalyseur se révèle très efficace pour ladite utilisation, car il se révèle être particulièrement stable, même en présence de charges à traiter contenant une grande quantité d'aromatiques lourds AC₉⁽⁺⁾

Les conditions opératoires pour ladite utilisation sont généralement les suivantes : une température comprise entre 250 et 650°C et de préférence entre 380 et 550°C ; une pression comprise entre 1 et 6 MPa et de préférence entre 2 et 4,5 MPa ; une vitesse spatiale d'alimentation, exprimée en kilogramme de charge introduite par kilogramme de catalyseur et par heure, comprise entre 0,1 et 10 h⁻¹ et de préférence entre 0,5 et 4 h⁻¹ ; un rapport molaire hydrogène sur hydrocarbures compris entre 2 et 20 et de préférence entre 3 et 12.

Les exemples suivants illustrent l'invention sans toutefois en limiter la portée.

### Exemple 1: Préparations de la zéolithe H-NU-87/1 selon l'invention

La matière première utilisée est une zéolithe NU-87, qui possède un rapport atomique Si/Al global égal à 17,2, une teneur pondérale en sodium correspondant à un rapport atomique Na/Al égal à 0,144. Cette zéolithe Nu-87 a été synthétisée d'après la demande de brevet européen EP-A-0.377.291.

Cette zéolithe NU-87 subit tout d'abord une calcination dite sèche à 550°C sous flux d'air sec durant 6 heures. Puis le solide obtenu est soumis à quatre échanges ioniques dans une solution de NH₄NO₃ 10N, à environ 100°C pendant 4 heures pour chaque échange. Le solide ainsi obtenu est référencé NH4-NU-87 et posséde un rapport Si/Al = 17,4 et un rapport Na/Al=0,002. Ses autres caractéristiques physicochimiques sont regroupées dans le tableau 1.

**Tableau 1**

| Echantillon | Diffraction X | | | | | | Adsorption | |
|---|---|---|---|---|---|---|---|---|
| | Paramètres | | | | | Cristallinité | ^{S}BET | V(P/Po=0,19) |
| | a (Å) | b (Å) | c (Å) | β (°) | V (Å³) | (%) | (m2/g) | ml N2 liquide/g |
| NH4-NU-87 | 14,35 | 22,34 | 25,14 | 151,53 | 3840 | 100 | 466 | 0,19 |

La zéolithe NH4-NU-87 est alors soumise à un traitement hydrothermique, en présence de 100% de vapeur d'eau à 625°C, durant 4 heures. La zéolithe subit alors une attaque acide, à l'aide d'acide nitrique 8 N, à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe NU-87 sèche (V/P=10).

A l'issue de ces traitements, la zéolithe H-NU-87/1 sous forme H posséde un rapport Si/Al atomique global égal à 28,8 et un rapport Na/Al<0,002. Ces caractéristiques cristallographiques et d'adsorption sont reportées dans le tableau 2, ci-après.

**Tableau 2**

| Echantillon | Diffraction X | | | | | | Adsorption | |
|---|---|---|---|---|---|---|---|---|
| | Paramètres | | | | | Cristallinité | ^{S}BET | V(P/Po=0,19) |
| | a (Å) | b (Å) | c (Å) | β (°) | V (Å³) | (%) | (m2/g) | ml N2 liquide/g |
| H-NU-87/1 | 14,34 | 22,29 | 25,13 | 151,5 | 3825 | 98 | 366 | 0,16 |

Ce tableau montre qu'après les étapes de steaming et d'attaque acide la zéolithe NU-87 conserve une bonne cristallinité et une surface spécifique (SBET) encore relativement élévée.

### Exemple 2 : Préparation du catalyseur C1 conforme à l'invention

La zéolithe H-NU-87/1 obtenue à l'exemple 1 est ensuite mise en forme par extrusion avec un gel d'alumine de manière à obtenir, après séchage et calcination sous air sec, le catalyseur C1 qui contient en poids 70 % de zéolithe H-NU-87/1 et 30 % d'alumine.

### Exemple 3 : Préparation du catalyseur C2 conforme à l'invention

Dans cet exemple, le catalyseur C1 préparé dans l'exemple 2, est soumis à 3 échanges ioniques avec une solution d'acétate de nickel de manière à introduire environ 1,0% poids de nickel dans le catalyseur.

Pour cela, le catalyseur C1 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est, pour chaque échange, ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur C2 ainsi obtenu contient, en % poids, 69,40 % de zéolithe H-NU-87/1, 29,75 % d'alumine et 0,85 % de nickel.

### Exemple 4 : Préparation de la zéolithe H-NU-87/2 conforme à l'invention

La matière première utilisée est la même zéolithe NU-87 que celle utilisée dans l'exemple 1. Cette zéolithe NU-87 subit, tout d'abord, une calcination dite sèche à 550°C sous flux d'air et d'azote durant 6 heures. Puis, le solide obtenu est soumis à un échange ionique dans une solution de NH₄NO₃ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires.

A l'issue de ces traitements, la zéolithe obtenue est référencée H-NU-87/2. Elle se trouve sous sa forme H et possède un rapport Si/Al atomique global égal à 34,6, et un rapport Na/Al égal à 0,007. Ces caractéristiques cristallographiques et d'adsorption sont reportées dans le tableau 3, ci-après.

**Tableau 3**

| Echantillon | Diffraction X | | | | | | Adsorption | |
|---|---|---|---|---|---|---|---|---|
| | Paramètres | | | | | Cristallinité | ^{S}BET | V(P/Po=0,19) |
| | a (Å) | b (Å) | c (Å) | β (°) | V (Å³) | (%) | (m2/g) | ml N2 liquide/g |
| H-NU-87/2 | 14,33 | 22,35 | 25,09 | 151,5 | 3835 | 97 | 496 | 0,21 |

### Exemple 5 : Préparation du catalyseur C3 conforme à l'invention

La zéolithe H-NU-87/2 obtenue à l'exemple 4 est ensuite mise en forme par extrusion avec un gel d'alumine de manière à obtenir, après séchage et calcination sous air sec, le catalyseur C3 qui contient en poids 70 % de zéolithe H-NU-87/2 et 30 % d'alumine.

### Exemple 6 : Préparation du catalyseur C4 non conforme à l'invention

La zéolithe NU-87 utilisée dans cet exemple est la zéolithe NH4-NU-87 préparée dans l'exemple 1 de la présente invention. Néanmoins, dans le présent exemple la zéolithe NU-87 ne subit pas de désalumination.

La zéolithe NH4-NU-87 obtenue à l'exemple 1 est ensuite mise en forme par extrusion avec un gel d'alumine de manière à obtenir, après séchage et calcination sous air sec, le catalyseur C4 qui contient en poids 70 % de zéolithe H-NU-87 et 30 % d'alumine.

Exemple 7 Evaluation des propriétés catalytiques en dismutation du toluène des catalyseurs C1, C2, C3, conformes à l'invention et du catalyseur C4 non conforme à l'invention

Les évaluations catalytiques sont réalisées en lit fixe, sous une pression totale de 2 MPa et un rapport molaire H2/toluène de 4. La charge utilisée est du toluène.

Dans la série de tests catalytiques dont les résultats sont regroupés dans le tableau 4, la pph est maintenue constante, c'est à dire que la vélocité spatiale de la charge, le toluène, exprimée en gramme de toluène injecté par gramme de catalyseur et par heure est identique dans les 4 tests. La température de réaction est ajustée de manière à obtenir un taux de conversion d'environ 49% poids en régime stabilisé.

**Tableau 4**

| Catalyseurs | C1 | C2 | C3 | C4 |
|---|---|---|---|---|
| T (°C) | 402 | 399 | 400 | 424 |
| Conv Toluène (% poids) | 48,85 | 48,92 | 49,05 | 48,96 |

Ces résultats mettent en évidence que les catalyseurs C1, C2 et C3 conformes à l'invention présentent une activité plus forte que le catalyseur non conforme C4.

Dans une autre série de tests catalytiques, la température de réaction est maintenue constante à 399°C, et la pph est ajustée de manière à maintenir une conversion d'environ 49% poids. Le tableau 5, ci-après, regroupe les sélectivités obtenues pour les différents catalyseurs.

**Tableau 5**

| Catalyseurs/Sélectivités (% poids) | C1 | C3 | C4 |
|---|---|---|---|
| C1-C4 | 0,5 | 0,6 | 0,6 |
| Benzène | 44,9 | 45,0 | 44,6 |
| Ethylbenzène | 0,5 | 0,4 | 0,4 |
| Xylènes | 47,5 | 47,4 | 46,9 |
| Aromatiques C9+ | 6,6 | 6,6 | 7,5 |

Les résultats du tableau 5 montrent que les catalyseurs conformes à l'invention, C1 et C3 sont plus sélectifs que le catalyseur C4 non conforme. En particulier, ils présentent des sélectivités en aromatiques lourds AC₉⁺, qui sont des produits indésirables, plus faibles que le catalyseur C4, non conforme à l'invention.

## Revendications

1. Zéolithe modifiée de type structural NES, comprenant du silicium et au moins un élément T choisi dans le groupe formé par l'aluminium, le fer, le gallium, et le bore, **caractérisée en ce qu'**une partie au moins d'élément T a été extraite de la charpente à partir de la zéolithe de type structural NES brute de synthèse et **en ce qu'**elle possède un rapport Si/T atomique global supérieur à 25.

2. Zéolithe selon la revendication 1 **caractérisée en ce qu'**elle a été désaluminée.

3. Zéolithe selon l'une des revendications 1 ou 2 telle que ledit élément T est l'aluminium.

4. Zéolithe Nu-87 selon l'une des revendications 1 à 3.

5. Zéolithe selon l'une des revendications 1 à 4 telle que le rapport molaire Si/T est compris entre 25 et 350.

6. Zéolithe selon l'une des revendications 1 à 5 telle que le rapport molaire Si/T est compris entre 25 et 150.

7. Préparation de la zéolithe selon l'une des revendications 1 à 6 à partir de la zéolithe brute de synthèse comprenant du structurant organique, par la méthode d'attaque acide directe.

8. Préparation de la zéolithe selon l'une des revendications 1 à 6 à partir de la zéolithe brute de synthèse comprenant du structurant organique, par la méthode de traitement thermique et d'attaque acide.

9. Préparation selon la revendication 8 telle que le traitement thermique est effectué en présence de vapeur d'eau.

10. Préparation de la zéolithe selon l'une des revendications 1 à 6 à partir de la zéolithe brute de synthèse comprenant du structurant organique par un composé chimique désaluminant.

11. Support de catalyseur comprenant au moins une matrice et au moins une zéolithe selon l'une des revendications 1 à 6 ou préparée selon l'une des revendications 7 à 10.

12. Support selon la revendication 11 dans lequel la matrice est choisie parmi les éléments du groupe formé par les argiles, la magnésie, les alumines, les silices, l'oxyde de titane, l'oxyde de bore, la zircone, les phosphates d'aluminium, les phosphates de titane, les phosphates de zirconium, les silice-alumines et le charbon.

13. Catalyseur comprenant au moins une matrice et au moins une zéolithe selon l'une des revendications 1 à 6 ou préparée selon l'une des revendications 7 à 10, au moins en partie sous forme acide.

14. Catalyseur selon la revendication 13 comportant en outre au moins un élément des groupes lB et VIII de la Classification Périodique des Eléments.

15. Catalyseur selon la revendication 14 tel que ledit élément est choisi dans le groupe formé par Ag, Ni et Pt.

16. Catalyseur selon l'une des revendications 13 à 15 tel que la matrice est choisie parmi les éléments du groupe formé par les argiles, la magnésie, les alumines, les silices, l'oxyde de titane, l'oxyde de bore, la zircone, les phosphates d'aluminium, les phosphates de titane, les phosphates de zirconium, les silice-alumines et le charbon.

17. Catalyseur selon l'une des revendications 13 à 16 comprenant de 10 à 99 % de zéolithe.

18. Catalyseur selon l'une des revendications 13 à 17 comprenant de 20 à 95 % de zéolithe.

19. Catalyseur selon l'une des revendications 14 à 18 comprenant de 0,01 à 10 % d'au moins un élément des groupes lB et VIII.

20. Préparation du catalyseur selon l'une des revendications 13 à 19 par mélange de la matrice et de la zéolithe puis par mise en forme suivie de l'éventuelle introduction dudit élément des groupes IB et VIII.

21. Utilisation d'un catalyseur selon l'une des revendications 13 à 19 ou préparé selon la revendication 20 pour la conversion d'hydrocarbures.

22. Utilisation selon la revendication 21 en dismutation d'hydrocarbures alkylaromatiques et/ou en transalkylation d'hydrocarbures alkylaromatiques.

23. Utilisation selon la revendication 22 en transalkylation du toluène et d'aromatiques comportant au moins 9 atomes de carbone par molécule, pouvant contenir de 0 à 100% d'hydrocarbures aromatiques à au moins 9 atomes de carbone par molécule par rapport au mélange total.

24. Utilisation selon l'une des revendications 22 ou 23 en dismutation du toluène.

## Patentansprüche

1. Modifizierter Zeolith vom NES Strukturtyp, Silizium und wenigstens ein Element T umfassend, das aus der durch Aluminium, Eisen, Gallium und Bor gebildeten Gruppe gewählt ist, **dadurch gekennzeichnet, dass** wenigstens ein Teil des Elements T aus dem Gerüst ausgehend vom Zeolith der rohen NES Synthesisstrukturtyp extrahiert wurde, und dass er ein Si/T Atomgesamtverhältnis von mehr als 25 besitzt.

2. Zeolith nach Anspruch 1, **dadurch gekennzeichnet, dass** er entaluminiert wurde.

3. Zeolith nach einem der Ansprüche 1 oder 2, derart, dass dieses Element T Aluminium ist.

4. Nu-87 Zeolith nach einem der Ansprüche 1 bis 3.

5. Zeolith nach einem der Ansprüche 1 bis 4, derart, dass das Molverhältnis Si/T zwischen 25 und 350 beträgt.

6. Zeolith nach einem der Ansprüche 1 bis 5, derart, dass das Molverhältnis Si/T zwischen 25 und 150 liegt.

7. Herstellung des Zeolith nach einem der Ansprüche 1 bis 6, ausgehend von dem rohen Synthesezeolith, einen organischen Strukturbildner umfassend, nach dem Verfahren des direkten Säureangriffs.

8. Herstellung des Zeolith nach einem der Ansprüche 1 bis 6, ausgehend von dem rohen Synthesezeolith, einen organische Strukturbildner umfassend, nach dem Verfahren der thermischen Behandlung sowie des Säureangriffs.

9. Herstellung nach Anspruch 8, derart, dass die thermische Behandlung in Anwesenheit von Wasserdampf durchgeführt wird.

10. Herstellung des Zeolith nach einem der Ansprüche 1 bis 6, ausgehend vom rohen Synthesezeolith, einen organischen Strukturbildner umfassend, mittels einer entaluminisierenden chemischen Verbindung.

11. Katalysatorträger, umfassend: wenigstens eine Matrix und wenigstens einen Zeolith nach einem der Ansprüche 1 bis 6 oder hergestellt nach einem der Ansprüche 7 bis 10.

12. Katalysatorträger nach Anspruch 11, bei dem die Matrix gewählt ist aus den Elementen der Gruppe, die durch die Tone, Mangesiumoxid oder die Aluminiumoxide, die Siliziumoxide, das Titanoxid, das Boroxid, das Zirkonoxid, die Aluminiumphosphate, die Titanphosphate, die Zirkoniumphosphate, die Siliziumoxide-Aluminiumoxide und Kohle gebildet ist.

13. Katalysator umfassend wenigstens eine Matrix und wenigstens einen Zeolith nach einem der Ansprüche 1 bis 6 oder hergestellt nach einem der Ansprüche 7 bis 10, zum Teil wenigstens in der sauren Form.

14. Katalysator nach Anspruch 13, im übrigen wenigstens ein Element der Gruppen IB und VIII des Periodensystems der Elemente umfassend.

15. Katalysator nach Anspruch 14, derart, dass das Element gewählt ist aus der durch Ag, Ni und Pt gebildeten Gruppe.

16. Katalysator nach einem der Ansprüche 13 bis 15, derart, dass die Matrix gewählt ist aus den Elementen der Gruppe, die gebildet ist durch die Tone, Magnesiumoxid, Aluminiumoxide, Siliziumoxide, Titanoxid, Boroxid, Zirkonoxid, Aluminiumphosphate, Titanphosphate, Zirkoniumphosphate, Siliziumoxid, Aluminiumoxide und Kohle.

17. Katalysator nach einem der Ansprüche 13 bis 16, 10 bis 99% Zeolith umfassend.

18. Katalysator nach einem der Ansprüche 13 bis 17, 20 bis 95% Zeolith umfassend.

19. Katalysator nach einem der Ansprüche 14 bis 18, zwischen 0,01 und 10% wenigstens eines Elements der Gruppen IB und VIII umfassend.

20. Herstellung des Katalysators nach einem der Ansprüche 13 bis 19 durch Mischen der Matrix und des Zeolith, dann durch Formgebung, gefolgt von einem eventuellen Zuführen dieses Elements der Gruppen IB und VIII.

21. Verwendung eines Katalysators nach einem der Ansprüche 13 bis 19 oder hergestellt nach Anspruch 21 zur Umwandlung der Kohlenwasserstoffe.

22. Verwendung nach Anspruch 21 zur Dismutation von alkylaromatischen Kohlenwasserstoffen und/oder zur Transalkylierung von alkylaromatischen Kohlenwasserstoffen.

23. Verwendung nach Anspruch 22 zur Transalkylierung des Toluols und von Aromaten, wenigstens 9 Kohlenstoffatomen pro Molekül umfassend, die zwischen 0 und 100% aromatische Kohlenwasserstoffe mit wenigstens 9 Kohlenstoffatomen pro Molekül bezogen auf das Gesamtgemisch enthalten können.

24. Verwendung nach einem der Ansprüche 22 oder 23 zur Dismutation des Tuluols.

## Claims

1. A modified zeolite with structure type NES, comprising silicon and at least one element T selected from the group consisting of aluminium, iron, gallium, and boron, **characterized in that** at least a portion of element T has been extracted from the framework of the as-synthesized zeolite with structure type NES and **in that** said modified zeolite has a global atomic ratio Si/T higher than 25.

2. A zeolite according to claim 1, **characterized in that** it has been dealuminated.

3. A zeolite according to claim 1 or claim 2, in which said element T is aluminium.

4. A NU-87 zeolite according to any one of claims 1 to 3.

5. A zeolite according to any one of claims 1 to 4, in which the Si/T molar ratio is in in the range 25 to 350.

6. A zeolite according to any one of claims 1 to 5, in which the Si/T molar ratio is in the range 25 to 150.

7. Preparation of a zeolite according to any one of claims 1 to 6 from as-synthesized zeolite containing an organic structuring agent, using the direct acid attack method.

8. Preparation of a zeolite according to any one of claims 1 to 6 from as-synthesized zeolite containing an organic structuring agent, using a heat treatment and acid attack method.

9. Preparation of a zeolite according to claim 8, in which the heat treatment is carried out in the presence of steam.

10. Preparation of a zeolite according to any one claims 1 to 6 from as-synthesized zeolite containing an organic structuring agent, using a chemical dealluminating compound.

11. A catalyst support comprising at least one matrix and at least one zeolite according to any one of claims 1 to 6, or prepared according to any one of claims 7 to 10.

12. A catalyst support according to claim 11, in which the matrix is selected from the group formed by clays, magnesia, aluminas, silicas, titanium oxide, boron oxide, zirconia, aluminium phosphates, titanium phosphates, zirconium phosphates, silica-aluminas and charcoal.

13. A catalyst comprising at least one matrix and at least one zeolite according to any one of claims 1 to 6, or prepared in accordance with any one of claims 7 to 10, at least partially in its acid form.

14. A catalyst according to claim 13, further comprising at least one element from groups IB and VIII of the periodic table.

15. A catalyst according to claim 14, in which said element is selected from the group formed by Ag, Ni and Pt.

16. A catalyst according to any one of claims 13 to 15, in which the matrix is selected from the group formed by clays, magnesia, aluminas, silicas, titanium oxide, boron oxide, zirconia, aluminium phosphates, titanium phosphates, zirconium phosphates, silica-aluminas and charcoal.

17. A catalyst according to any one of claims 13 to 16, comprising 10% to 99% of zeolite.

18. A catalyst according to any one of claims 13 to 16, comprising 20% to 95% of zeolite.

19. A catalyst according to any one of claims 14 to 18, comprising 0.01% to 10% of at least one element from groups IB and VIII.

20. Preparation of a catalyst according to any one of claims 13 to 19 by mixing matrix and a zeolite then forming, followed by optional introduction of said element from groups IB and VIII.

21. Use of a catalyst according to any one of claims 13 to 19 or prepared according to claim 21, for the conversion of hydrocarbons.

22. Use according to claim 21 for the dismutation of alkylaromatic hydrocarbons and/or transalkylation of alkylaromatic hydrocarbons.

23. Use according to claim 22 for the transalkylation of toluene and aromatic hydrocarbons comprising at least 9 carbon atoms per molecule, which may contain 0 to 100% of aromatic hydrocarbons containing at least 9 carbon atoms per molecule with respect to the total mixture.

24. Use according to any one of claims 22 or 23 for the dismutation of toluene.
